# EUROPEAN PATENT SPECIFICATION

(11) **EP 1 060 759 B1**
(45) Date of publication and mention of the grant of the patent: **31.03.2004**
(21) Application number: 00650069.8
(22) Date of filing: 13.06.2000
(51) Int. Cl.: A61M 25/10, A61M 25/00, A61L 29/06

(54) **Medical balloon catheter having an inflation tube with a polyetherimide segment**
Medizinischer Katheter mit einem Schlauch der abschnittsweise aus Polyetherimide besteht zum Auffüllen eines Ballons
Cathéter médical à ballon ayant un tube de gonflage en partie en polyétherimide

(30) Priority: 15.06.1999 US 333345
(43) Date of publication of application: 20.12.2000
(73) Proprietor: Medtronic, Inc., Minneapolis, MN 55432 (US)
(72) Inventor: WU, Pin-pin, Santa Rosa, California 95409 (US); MORENO, Juan, Wildomar, California 92595 (US); CHEN, Ziyun, San Diego, California 92128 (US); DAVISON, Paul O., Poway, California 92064 (US); TRAN, Don. H., Westminister, California 92683 (US)
(74) Representative: McKeown, Yvonne Mary

(56) References cited:
- EP-A- 0 462 482
- US-A- 5 100 381
- US-A- 5 810 867

## Description

### FIELD OF THE INVENTION

The present invention pertains generally to inflation tubes for medical catheters and methods for their manufacture. More specifically, the present invention pertains to an inflation tube for a balloon catheter which incorporates segments that are made of different materials in order for the catheter to exhibit the beneficial characteristics of both materials. The present invention is particularly, but not exclusively, useful as an inflation tube having a proximal segment which is made of a polyetherimide (PEI) material, to provide sufficient stiffness for the inflation tube while retaining as much flexibility as possible in the proximal segment.

### BACKGROUND OF THE INVENTION

Medical balloon catheters commonly include an inflation tube and a catheter balloon that is attached in fluid communication to one end of the inflation tube. In use, the balloon catheter is advanced into the patient's vasculature to position the balloon at the therapy site. Once the balloon is in position, an inflator pumps saline solution through the inflation tube to inflate the balloon and thereby dilate a stenosis at the therapy site.

For the proper operation of a balloon catheter, the inflation tube should exhibit several specific characteristics. These characteristics include: stiffness, to provide good "pushability", for advancement of the catheter into the vasculature without kinking the inflation tube; and flexibility, to provide good "steerability", for maneuvering the inflation tube through the patient's vasculature enroute to the therapy site.

While stiffness and flexibility are both important characteristics for the inflation tube of a balloon catheter, their relative importance varies along the length of the inflation tube. Specifically, stiffness tends to be more important in the proximal segment of the inflation tube, while flexibility is usually more important in the distal segment of the tube. Further, these material characteristics are contrariwise to each other. Consequently, to maximize both stiffness and flexibility for the entire inflation tube, two segments that are made of different materials may need to be considered for the manufacture of the tube. One material is chosen for the proximal segment to provide the required stiffness for pushability in the inflation tube. The other material for the distal segment is selected to provide the required flexibility for steerability of the inflation tube.

It should be kept in mind that stiffness and flexibility are important for the entire inflation tube. Accordingly, although the proximal segment emphasizes stiffness, the proximal segment should retain as much flexibility as is feasible. To determine the suitability of a material for the proximal segment, stiffness and flexibility can be evaluated in terms of material parameters. Specifically, a material with good compressive strength provides a proximal segment with good stiffness for pushability. Similarly, flexural strength, or the resistance of a material to bending, is an indicator of flexibility. Flexibility varies in such a way that as the flexural strength of a material increases, its flexibility decreases. An optimal proximal segment is therefore made of a material which is strong in compression, to emphasize stiffness, but which has an appropriate flexural strength for flexibility. Stated differently, although the material for the proximal segment should have good flexural strength, the material should be stronger in compression than in flexure. As an overlying consideration, the materials for the proximal and distal segments must be thermally compatible with each other, for thermal bonding. Thermal bonding of the tube segments yields an integral inflation tube and prevents leakage in the inflation tube where the segments are joined.

For a segmented inflation tube, proximal segments which are made of a material having good stiffness for pushability have been disclosed in the prior art. For example, U.S. Patent 5,569,196, which issued to Ainsworth et al. for an invention entitled "Intraluminal Catheter With High Strength Proximal Shaft", discloses an inflation tube in which a proximal section that is made of a stiff polyetheretherketone (PEEK) material is glued to a distal section that is made of a softer material. A PEEK material, however, is inherently quite strong in flexure (i.e. it is very stiff). Thus, a proximal section for an inflation tube as disclosed by Ainsworth et al. accepts excessive stiffness in order to achieve sufficient pushability for the inflation tube. Further, an inflation tube manufactured in accordance with Ainsworth et al. is susceptible to separation (and leakage) where the segments are joined. Furthermore, for example US-A-5 810 867 discloses a dilation cathether having a balloon inflation shaft, comprising a stiff proximal section made of polyimide and a relatively flexible distal section, both sections joined adhesively.

In light of the above, it is an object of the present invention to provide a two-segment inflation tube for a balloon catheter in which the proximal segment of the tube is made of a material that has good flexibility while providing the column strength that is necessary for pushability. It is another object of the present invention to provide an inflation tube with a relatively stiff proximal segment that is made of a material which can be thermally bonded with a relatively soft, flexible material for its companion distal segment. Still another object of the present invention is to provide an inflation tube for a balloon catheter which is effectively easy to use, relatively simple to manufacture and comparatively cost effective.

### SUMMARY OF THE PREFERRED EMBODIMENTS

A two-segment inflation tube in accordance with the present invention exhibits both of the desired functional characteristics of a catheter, namely: stiffness and flexibility. To do this, the inflation tube includes a proximal segment which has relatively increased stiffness when compared with its companion distal segment. Although stiffness is the primary concern in the proximal segment of the inflation tube, flexibility cannot be completely disregarded. Accordingly, although the proximal segment emphasizes stiffness for pushability of the inflation tube, the proximal segment retains as much flexibility as is feasible.

For the manufacture of the proximal segment of the present invention, a polyetherimide (PEI) material is chosen. This PEI material provides stiffness for pushability for the inflation tube. To provide the stiffness for pushability, the PEI material is strong in compression. Specifically, the PEI material has a compressive strength, σ_{c}, substantially in the range between 20,000 and 24,000 pounds per square inch (20,000 psi < σ_{c} < 24,000 psi). The PEI material for a proximal segment also retains as much flexibility as is feasible. Flexibility can be described in terms of flexural strength, σ_{f}, or resistance to bending. For the proximal segment, the PEI material has a flexural strength, σ_{f}, substantially in the range between 18,000 and 22,000 pounds per square inch (18,000 psi < σ_{f} < 22,000 psi). In general, the flexibility of a material can also be described in terms of a material's flexural modulus, or the amount of deflection of a material under stress. The higher the flexural modulus, the less the material bends in response to a stress, and a high-flexural modulus material has less flexibility than a lower-flexural modulus material. For the PEI material of the proximal segment, the flexural modulus, FM, is substantially in the range between 450,000 and 550,000 pounds per square inch (450 ksi < TM < 550 ksi). Empirically, a material which has a FM in this range and the compressive and flexural strengths described above effectively performs as a material for the proximal segment. Finally, the PEI material for the proximal segment is thermally compatible with the material for the distal segment for thermal bonding to establish an integral inflation tube. In the preferred embodiment, the PEI material for the proximal segment is Ultem1000®. The chosen material is extruded in any manner known by the skilled artisan to form the proximal segment of the inflation tube.

The inflation tube includes a distal segment for providing sufficient flexibility for steerability of the inflation tube. For integration with the proximal segment, the proximal end of this distal segment is thermally bonded to the distal end of the proximal segment. More specifically, the proximal end of the distal segment is positioned to surround an intermediate region which is near the distal end of the proximal segment. Once in place, the proximal end of the distal segment is thermally bonded onto the intermediate region of the proximal segment. This establishes a unitary, integral inflation tube which has sufficient stiffness in the proximal segment and sufficient flexibility in the distal segment for effective operation of the catheter. For thermal bonding, the distal segment is preferably made of Polyether Block Amide (PEBA) co-polymer, such as Pebax®7233, a material which is thermally compatible with the Ultem1000® material for the proximal segment.

The catheter may also include a multi-layered guidewire tube and an inflatable tubular balloon. For thermal compatibility, the materials for the guidewire tube and the balloon are from the same general chemical family as the materials for the inflation tube. Accordingly, the outer layer of the guidewire tube and the balloon are preferably made of a nylon polymer and a PEBA material, respectively. For assembly, the guidewire tube is positioned within the inflation tube to extend distally therefrom. With the guidewire tube in this position, the balloon is attached to the outer layer of the guidewire tube and the inflation tube. Specifically, the balloon is positioned over the distal portion of the guidewire tube and the distal end of the balloon is thermally bonded to the distal end of the guidewire tube. The proximal end of the balloon is thermally bonded to the distal end of the inflation tube to place the balloon in fluid communication with the inflation tube. The result is a medical balloon catheter which incorporates the proximal inflation tube of the present invention.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a perspective view of the catheter according to a preferred embodiment of the present invention;
Figure 2 is an exploded perspective view of the catheter which shows the connective relationships between the component parts;
Figure 3 is a perspective view of the multi-layered guidewire tube with portions cut away for clarity;
Figure 4 is a cross-sectional view of the multi-layered guidewire tube when taken along the line 4 - 4 in Figure 3;
Figure 5 is a perspective view of the proximal inflation tube;
Figure 6 is a perspective view of the transition tube component;
Figure 7 is a cross-sectional view of the transition section of the assembled catheter which shows the transition tube in its operational environment (with the guidewire tube component omitted for clarity) when taken along line 7 - 7 in Figure 1;
Figure 8A is a cross-sectional view of the catheter when taken along the line 8A - 8A in Figure 7;
Figure 8B is a cross-sectional view of the catheter when taken along the line 8B - 8B in Figure 7;
Figure 8C is a cross-sectional view of the catheter when taken along the line 8C - 8C in Figure 7;
Figure 8D is a cross-sectional view of the catheter when taken along the line 8D - 8D in Figure 7; and
Figure 8E is a cross-sectional view of the catheter when taken along the line 8E - 8E in Figure 7.

### DESCRIPTION OF THE PREFERRED EMBODIMENT

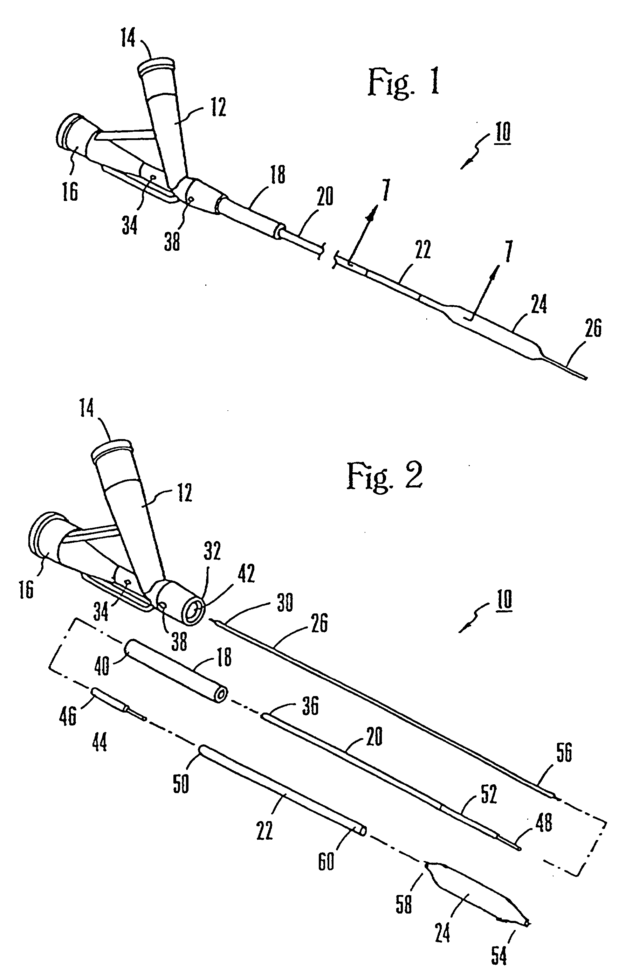

Referring initially to Fig. 1, a balloon catheter in accordance with a preferred embodiment of the present invention is shown and generally designated 10. In brief overview, and as shown in Fig. 1, the catheter 10 includes a manifold 12 which is formed with an inflation port 14 and a guidewire port 16. It can also be seen in Fig. 1, by progressing distally from the manifold 12, that the catheter 10 additionally includes a strain relief member 18, a proximal inflation tube 20, a distal inflation tube 22, a balloon 24, and a guidewire tube 26

In order to best appreciate how the components of the catheter 10 are interconnected, it is perhaps best to refer to Fig. 2. As shown in Fig. 2, the proximal end 30 of the guidewire tube 26 is inserted into the distal end 32 of the manifold 12. The proximal end 30 of the guidewire tube 26 is then bonded in fluid communication with the guidewire port 16 of the manifold 12. To do this, adhesives (not shown) are injected through injection port 34. The proximal inflation tube 20 is then positioned over the guidewire tube 26 to place the tubes 20, 26 in a coaxial arrangement, with the guidewire tube 26 being surrounded by the proximal inflation tube 20. The proximal end 36 of the proximal inflation tube 20 is then inserted into the distal end 32 of the manifold 12 and bonded with adhesives (not shown) which are injected through injection port 38. This bonding of the proximal inflation tube 20 to the manifold 12 places the proximal inflation tube 20 in fluid communication with the inflation port 14 of the manifold 12. Importantly, the guidewire tube 26 is not in fluid communication with the inflation port 14 when it is glued to the manifold 12. Similarly, the proximal inflation tube 20 is in fluid isolation from the guidewire port 16 when it is glued to the manifold 12. Next, the strain relief member 18 is slipped over the combination of the proximal inflation tube 20 and guidewire tube 26. An adhesive (not shown) is applied to the proximal end 40 of relief member 18, and the member 18 is placed into the socket 42 of the manifold 12 and bonded to the distal end 32 of the manifold 12.

The catheter 10 also includes a transition tube 44. As indicated in Fig. 2, the transition tube 44 is slipped over the guidewire tube 26, and the proximal end 46 of the transition tube 44 is thermally bonded to the distal region 48 of the proximal inflation tube 20. As implied in Fig. 2, the distal inflation tube 22 is positioned over the combination of the guidewire tube 26 and the transition tube 44. Next, the proximal end 50 of the distal inflation tube 22 is thermally bonded to an intermediate region 52 on the proximal inflation tube 20. Although the distal inflation tube 22 is not bonded to the transition tube 44, the distal inflation tube 22 completely covers the transition tube 44 once it is bonded to the proximal inflation tube 20. Further, the transition tube 44 is slidingly disposed within the distal inflation tube 22. Finally, the distal end 54 of the balloon 24 is thermally bonded to the distal end 56 of the guidewire tube 26, and the proximal end 58 of the balloon 24 is thermally bonded to the distal end 60 of the distal inflation tube 22. With this configuration, a path of fluid communication exists, in order, from the inflation port 14 of the manifold 12, through the proximal inflation tube 20, transition tube 44, distal inflation tube 22 and into the balloon 24.

It is to be noted at this point that the polymer materials which have been selected for the fabrication of the proximal inflation tube 20, the distal inflation tube 22, the balloon 24 and the transition tube 44 of the catheter 20 are thermally compatible with each other. Specifically, the selected polymer materials are thermally compatible with each other in the sense that the material for the proximal inflation tube 20, polyetherimide (PEI), is capable of being thermally bonded with a Polyether Block Amide (PEBA) co-polymer, which is the preferred material for distal inflation tube 22, the balloon 24 and the transition tube 44. This is important because, as illustrated in Fig. 2 and mentioned above, the intermediate region 52 of the proximal inflation tube 20 is to be thermally bonded to the distal inflation tube 22. Also, the transition tube 44 is to be thermally bonded to the distal region 48 of the proximal inflation tube. This thermal bonding can be accomplished in any manner known by the skilled artisan.

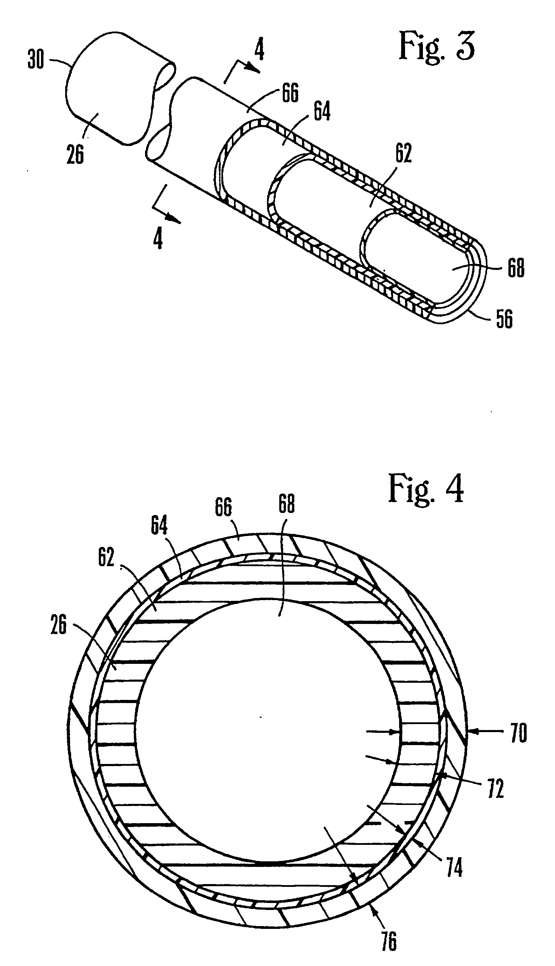

Referring now to Fig. 3, the multi-layered structure of the guidewire tube 26 is to be more fully appreciated. As shown in Fig. 3, the guidewire tube 26 has a tie layer 64 which is thermally bonded onto an inner layer 62, and an outer layer 66 is thermally bonded onto the tie layer 64. As also shown in Fig. 3, the guidewire tube 26 is formed with a guidewire lumen 68 for receiving a guidewire (not shown) therethrough. For free and easy movement of the guidewire (not shown) when engaged therewith, the inner layer 62 of the tube 26 is made of a lubricious material. Specifically, the material for the inner layer 62 preferably has a coefficient of friction with metal of less than 0.3 (C_{f} < 0.3). Further, the material for the inner layer 62 has a Shore Durometer stiffness reading which is substantially within a range of thirty to eighty Durometers (30D - 80D). To satisfy these parameters, the material for the inner layer 62 may be a polyethylene, a fluoropolymer or a polymer blended with another low-friction polymer or with a low-friction filler. In the preferred embodiment, the material for the inner layer 62 is High Density Polyethylene (HDPE).

The outer layer 66 of the guidewire tube 26 in Fig. 3 is made of a material which has a greater stiffness than the material for the inner layer 62, as measured by a Shore Durometer test. Further, the material for the outer layer 66 depends on the material for the balloon 24. More specifically, the material for the outer layer 66 is substantially from the same chemical family as the material for the balloon 24. Accordingly, since the preferred material for the balloon 24 is a Polyether Block Amide (PEBA) co-polymer, the preferred material for the outer layer 66 is a nylon polymer such as Vestamid®. In order to thermally bond the inner layer 62 to the outer layer 66, the material for the tie layer 64 is thermally compatible with the materials for both the inner layer 62 and the outer layer 66 for the guidewire tube 26. For this purpose, the material for tie layer 64 is softer than the materials for the respective inner and outer layers 62, 66, as measured by the Shore Durometer test. Accordingly, the preferred material for the tie layer 64 is a co- or ter-polymer which includes maleic anhydride, such as Lotader® resins which are manufactured by Elf Altochem®. With this configuration, the guidewire tube 26 has good lubricity qualities in the soft inner layer 62 for efficient guidewire engagement without sacrificing stiffness, which is provided by the stiff outer layer 66.

Referring now to Fig. 4, the cross-sectional dimensions of the guidewire tube 26 are to be appreciated. As shown in Fig. 4, the guidewire tube 26 has an overall thickness 70. The inner layer 62 of the guidewire tube 26 has an inner layer thickness 72, and this inner layer thickness 72 is preferably with a range of substantially twenty-five to sixty percent (25 - 60%) of the overall thickness 70 of the guidewire tube 26. To minimize the overall profile of the guidewire tube 26, the tie layer 64 has a tie layer thickness 74 which is preferably less than four percent of the overall thickness 70 of the tube 26. As shown in Fig. 4, the outer thickness 76 of the outer layer 66 forms the remainder of the overall thickness 70 of the guidewire tube 26. In the preferred embodiment, the materials are co-extruded in a manner well known in the prior art to achieve the desired respective thickness 72, 74, 76 for each respective layer 62, 64, 66. In this manner, the multi-layered guidewire tube 26 of the present invention is formed.

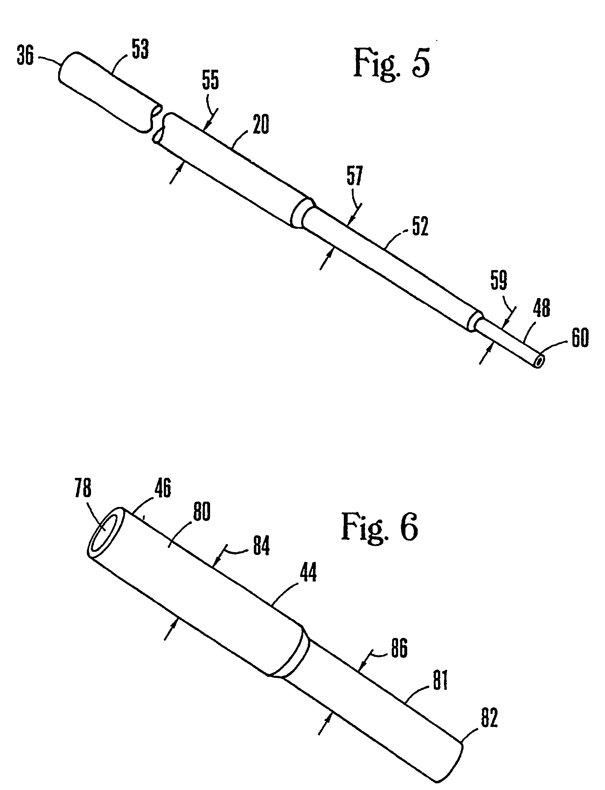

In Fig. 5, a perspective view of the proximal inflation tube 20 of the present invention is shown. As shown, the proximal inflation tube 20 is formed with a distal region 48 which is near the distal end 60 of the tube 20 and an intermediate region 52 which is immediately proximal to the distal region 48. Further, the proximal inflation tube 20 has a proximal region 53 which is adjacent the proximal end 36 of the tube 20. Importantly, the outside diameter 55 of the proximal region 53 is greater than the outside diameter 57 at the intermediate region 52. Similarly, the outside diameter 57 of the intermediate region 52 is greater than the outside diameter 59 of the distal region 48. With this configuration, a substantially uniform overall catheter profile is maintained when the transition tube 44 and distal inflation tube 22 (not shown in Fig. 5, see Fig. 2) are attached as discussed above.

The selection of a material for the manufacture of the proximal inflation tube 20 is important. Specifically, and as discussed above, the distal inflation tube 22 and the transition tube 44 (not shown in Fig. 5) are thermally bonded to the proximal inflation tube 20 during assembly of the catheter 10. For this purpose, the proximal inflation tube 20 is made of a material which is thermally compatible with the materials for the distal inflation tube 22 and the transition tube 44. It is also important that the proximal inflation tube 20 is made of a material which emphasizes stiffness, for good pushability in the tube 20. Although the material for the proximal inflation tube 20 emphasizes stiffness, the material retains as much flexibility for the proximal inflation tube 20 as possible. Empirically, the material for the proximal inflation tube 20 is a polyetherimide (PEI) material. This PEI material has a compressive yield strength, σ_{c}, substantially in the range between 20,000 pounds and 24,000 pounds per square inch (20,000 psi < σ_{c} < 24,000 psi). Further, the PEI material for the proximal inflation tube 20 has a flexural strength, σ_{f}, substantially in a range of 18,000 - 22,000 pounds per square inch (18,000 psi < σ_{f} < 22,000 psi). Additionally, the PEI material has a flexural modulus, FM, of substantially in the range of 450,000 to 550,000 pounds per square inch (450 ksi < FM < 550 ksi). In the preferred embodiment of the invention, the preferred PEI material is Ultem1000®.

Referring now to Fig. 6, the transition tube 44 can be seen in greater detail. As shown in Fig. 6, the transition tube 44 is formed with a lumen 78 and has a proximal region 80 near the proximal end 46 of the tube 44. Similarly, the transition tube 44 has a distal region 81 near the distal end 82 of the transition tube 44. As also shown in Fig. 6, the proximal region 80 has a proximal diameter 84, and this proximal diameter 84 is greater than the distal diameter 86 for the distal region 81. For an alternative embodiment, the transition tube 44 has a substantially uniform profile throughout the tube 44. Stated differently, the proximal diameter 84 for the proximal region 80 is equal to the distal diameter 86 for the distal region 81 for the alternative embodiment of the transition tube 44. For either embodiment, it is preferable that the transition tube 44 be made of a material which is thermally compatible with the PEI material for the proximal inflation tube 20. Accordingly, the transition tube 44 is made of a Polyether Block Amide (PEBA) co-polymer, such as Pebax®, in the preferred embodiment of the present invention.

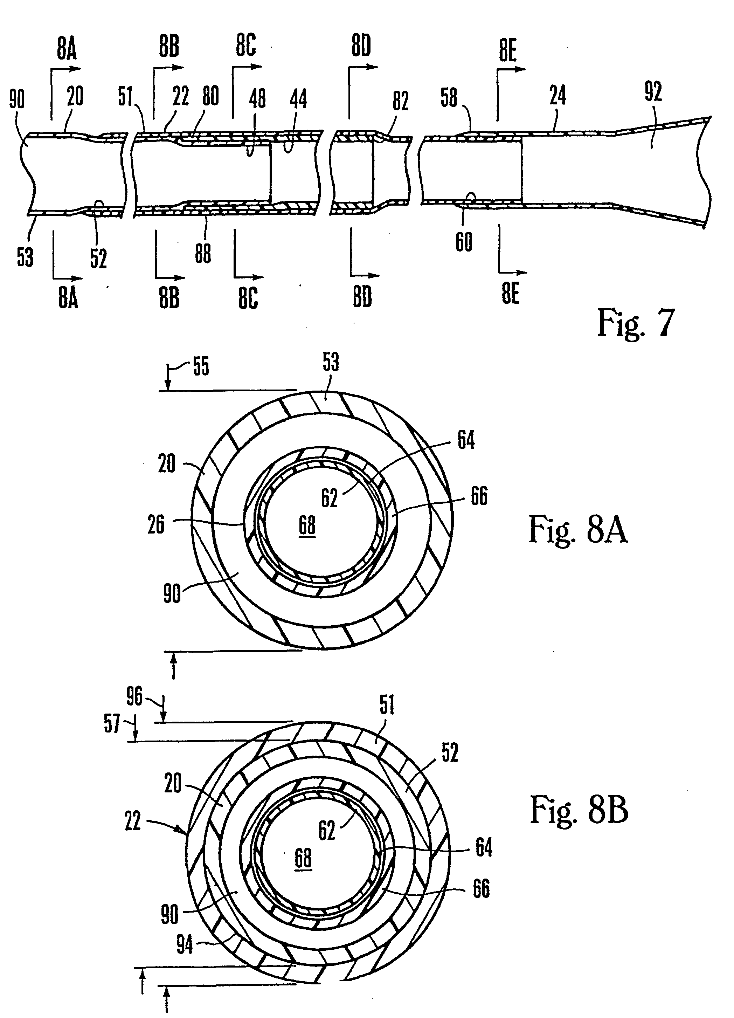

The cooperation of structure between the transition tube 44 and the proximal and distal tubes 20, 22 of the catheter 10 is to be more fully appreciated by referring to Fig. 7. For clarity, the guidewire tube has been omitted from Fig. 7. It is to be understood, however, that the guidewire tube is positioned in the inflation lumen 90 of the catheter 10 throughout the portion of the catheter 10 which is shown in Fig. 7. In Fig. 7, the intermediate and distal regions 52, 48 of the proximal inflation tube 20, the transition tube 44 and the proximal region 51 of the distal inflation tube collectively define a transition section 88 in the catheter 10. Specifically, the proximal region 80 of the transition tube 44 is positioned to surround the distal region 48 of the proximal inflation tube 20 and thermally bonded thereon. This places the transition tube 44 in fluid communication with the proximal inflation tube 20. Next, the distal inflation tube 22 is positioned to surround the combination of the transition tube 44 and the proximal inflation tube 20. Once in position, the proximal region 51 of the distal inflation tube 22 is thermally bonded to intermediate region 52 of the proximal inflation tube. Proceeding distally from the transition section 88, the proximal end 58 of the balloon 24 is thermally bonded to the distal end 60 of the distal inflation tube 22. With this configuration, a path of fluid communication (the inflation lumen 90) is established from, in proximal-to-distal order, the proximal inflation tube 20, through the transition tube 44 to the distal inflation tube 22 and into the interior 92 of the balloon 24.

The cooperation of structure at various points along the catheter 10 is to be more fully appreciated by cross-referencing Fig. 7 with Figs. 8A through Fig. 8E. Fig. 8A is a cross section of the catheter 10 which is taken at the proximal region 53 of the proximal inflation tube 20 which is shown in Fig. 7. (Note: In Figs. 8A-8E, the guidewire tube 26 of the present invention is included.) As shown in Fig. 8A, the guidewire lumen 68 is formed by the guidewire tube 26. The proximal inflation tube 20 surrounds the guidewire tube 26, and this arrangement creates an annular inflation lumen 90.

Fig. 8B shows a cross-section of the catheter 10 which is taken at the intermediate region 52 (shown in Fig. 7) of the proximal inflation tube 20. As shown in Fig. 8B, the distal inflation tube 22 is included in the cross-section of the catheter 10. Specifically, the proximal region 51 of the distal inflation tube 22 has been thermally bonded to the intermediate region 52 of the proximal inflation tube 20, as shown by thermal bond 94. Importantly, the outside diameter 96 of the distal inflation tube 22 (Shown in Fig. 8B) is substantially equal to the outside diameter 55 of the proximal inflation tube 20 (See Fig. 8A) after the tubes 20, 22 are thermally bonded. This is because the outside diameter 57 of the intermediate region 52 is less than the outside diameter 55 of the proximal region 51 of the proximal inflation tube 20 as described above (See Fig. 5). Thus, a substantially uniform overall profile is maintained in the catheter 10 when the proximal inflation tube 20 and distal inflation tube 22 are joined as discussed above.

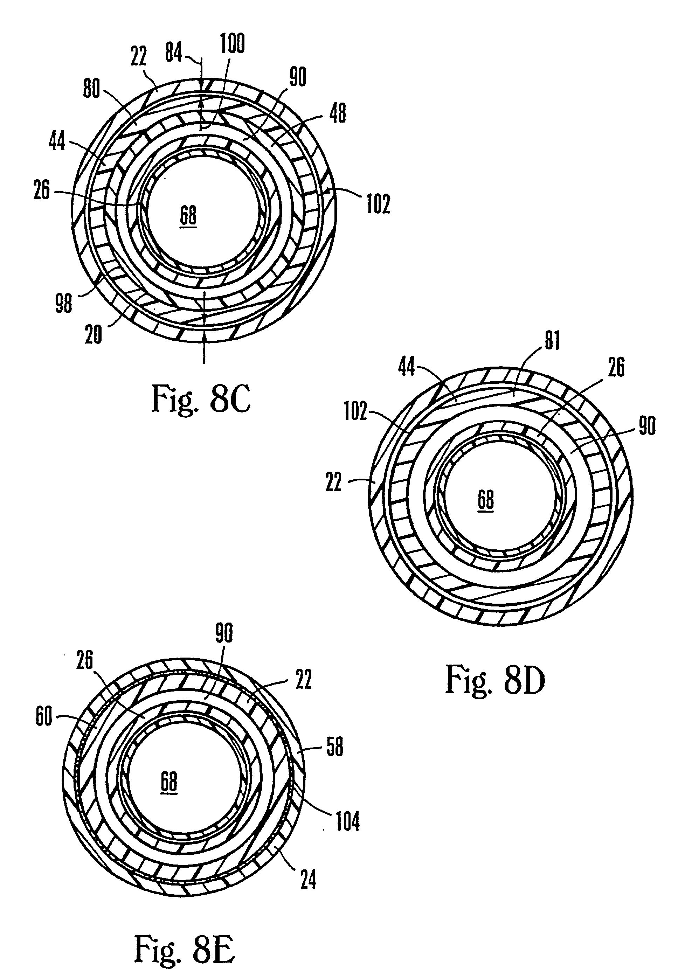

Fig. 8C is a cross section of the catheter 10 taken at the distal region 48 of the proximal inflation tube 20 which is shown in Fig. 7. As shown in Fig. 8C, the distal region 48 of the proximal inflation tube 20 surrounds the guidewire tube 26. Proceeding radially outward from the distal region 48, the proximal region 80 of the transition tube 44 is thermally bonded thereon, as indicated by thermal bond 98. As also shown in Fig. 8C, the distal inflation tube 22 surrounds the transition tube 44. The distal inflation tube 20, however, is not bonded to the transition tube 44. Instead, because the outside diameter 84 of the transition tube 44 is less than the inside diameter 100 of the distal inflation tube 22, a gap 102 is established between the transition tube 44 and the distal inflation tube 22. For an alternative embodiment, there is no gap 102 between the transition tube 44 and the distal inflation tube 22. Importantly, regardless whether there is a gap 102 between the transition tube 44 and distal inflation tube 22, there is no thermal bonding between the transition tube 44 and the distal inflation tube 22. With no thermal bonding, the transition tube 44 is slidingly disposed within the distal inflation tube 22. As a result, the combination of the transition tube 44 and proximal inflation tube 20 can move longitudinally relative to the distal inflation tube 22 when the catheter 10 bends in the transition section 88. Thus, the transition tube 44 provides for increased flexibility in the transition section 88 of the catheter 10. Additionally, the transition tube 44 longitudinally reinforces the transition section 88 of the catheter. Therefore, the transition tube 44 provides both stiffness and flexibility for the catheter of the present invention.

Fig. 8D is a cross-section of the assembled catheter 10 taken at the distal region 81 of the transition tube 44 which is shown in Fig. 7. In Fig. 8D, the distal region 81 of the transition tube 44 surrounds the guidewire tube 26 and forms the annular inflation lumen 90, as discussed above. As also discussed above, the gap 102 between the transition tube 44 and the distal inflation tube 22 is also shown in Fig. 8D. For this portion of the transition tube 44, however, the gap 102 exists for either embodiment of the tube 44. Even though the proximal inflation tube (not shown) has terminated, the transition tube 44 provides additional stiffness for the distal inflation tube 22 of the catheter 10. Fig. 8E is a cross-section of the assembled catheter 10 which is taken near the distal end 60 of the distal inflation tube 22 which is shown in Fig. 7. As indicated in Fig. 8E, the proximal end 58 of the balloon 24 has been thermally bonded to the distal end 60 of the distal inflation tube 22, as shown by thermal bond 104.

While the particular Medical Balloon Catheter Having An Inflation Tube With Integrated Segments as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

### Component List

- 10: catheter
- 12: manifold
- 14: inflation port
- 16: guidewire port
- 18: strain relief member
- 20: proximal inflation tube
- 22: distal inflation tube
- 24: balloon
- 26: guidewire tube
- 28 30: proximal end, guidewire tube
- 32.: distal end of manifold
- 34: injection port
- 36: proximal end of proximal inflation tube
- 38: injection port
- 40: proximal end of strain relief member
- 42: manifold socket
- 44: transition tube
- 46: proximal end of transition tube
- 48: distal region of proximal inflation tube
- 50: proximal end of distal inflation tube
- 51: proximal region of distal inflation tube
- 52: intermediate region of proximal inflation tube
- 53: proximal region of proximal inflation tube
- 54: balloon distal end
- 55: outside diameter of proximal region of proximal inflation tube
- 56: guidewire tube distal end
- 57: outside diameter of intermediate region of proximal inflation tube
- 58: balloon proximal end
- 59: outside diameter of distal region of proximal inflation tube
- 60: distal end of distal inflation tube
- 62: inner layer
- 64: tie layer
- 66: outer layer
- 68: guidewire lumen
- 70: overall tube thickness
- 72: inner layer thickness
- 74: tie layer thickness
- 76: outer layer thickness
- 78: transition tube lumen
- 80: transition tube proximal region
- 81: transition tube distal region
- 82: transition tube distal end
- 84: outside diameter of proximal region of transition tube
- 86: outside diameter of distal region of transition tube
- 88: transition section
- 90: inflation lumen
- 92: balloon interior
- 94: thermal bond
- 96: outside diameter of proximal region of distal inflation tube
- 98: thermal bond
- 100: inside diameter of transition tube
- 102: gap
- 104: thermal bond

## Claims

1. An inflation tube for providing stiffness and flexibility for a medical balloon catheter, said inflation tube comprising:
a proximal segment (20) having a proximal end (36) and a distal end (48) and being formed with a lumen, said proximal segment being made of a polyetherimide material for providing stiffness for said catheter; and
a distal segment (22) having a proximal end (50) and a distal end (60) and being made of a material for providing flexibility for said catheter, said material of said distal segment being thermally compatible with said polyetherimide material, said proximal end of said distal segment being thermally bonded in fluid communication to said distal end of said proximal segment.

2. An inflation tube as recited in claim 1 wherein said proximal segment and said distal segment, in combination, establish an inflation lumen (90) for said inflation tube and further comprising:
a guidewire tube (26) having a proximal end and a distal end, said guidewire tube being positioned in said inflation lumen to extend distally from said distal segment; and
an inflatable tubular balloon (24) having a proximal end and a distal end, said distal end of said balloon being attached to said distal end of said guidewire tube, said proximal end of said balloon being attached to said distal end of said distal segment to place said balloon in fluid communication with said inflation tube.

3. An inflation tube as recited in claim 1 wherein said polyetherimide material is stronger in compression than in flexure.

4. An inflation tube as recited in claim 1 wherein said polyetherimide material has a compressive yield strength substantially in the range between 20,000 and 24,000 pounds per square inch (20000 psi < σ_{c} < 24000 psi).

5. An inflation tube as recited in claim 4 wherein said polyetherimide material has a flexural strength substantially in the range between 18,000 and 22,000 pounds per square inch (18,000 psi < σ_{f} < 22,000 psi).

6. An inflation tube as recited in claim 5 wherein said polyetherimide material has a flexural modulus substantially in the range between 450,000 and 550,000 pounds per square inch (400 ksi < FM < 550 ksi).

7. An inflation tube as recited in claim 6 wherein said PEI material is Ultem1000®.

8. An inflation tube as recited in claim 2 where respective guidewire tube and said balloon are made of respective materials, said respective materials for said guidewire tube and for said balloon being thermally compatible with said polyetherimide material and with said material for said distal segment.

9. An inflation tube as recited in claim 8 wherein said balloon is thermally bonded to said guidewire tube and to said distal end of said distal segment.

10. A medical balloon catheter comprising:
a proximal inflation tube (20) having a proximal end (36) and a distal end (48) and being made from a polyetherimide material for providing stiffness for said catheter;
a distal inflation tube (22) made of a material for providing flexibility in said catheter and having a proximal end (50) and a distal end (60), said material for said distal inflation tube being thermally compatible with said polyetherimide material, said proximal end of said distal inflation tube being thermally bonded in fluid communication to said distal end of said proximal inflation tube to establish an inflation lumen;
a guidewire tube (26) having a proximal end and a distal end and being formed with a guidewire lumen (68), said guidewire tube being positioned in said inflation lumen to extend distally from said distal inflation tube; and
an inflatable tubular balloon (24) having a proximal end and a distal end, said distal end of said balloon being attached to said distal end of said guidewire tube, said proximal end of said balloon being attached to said distal end of said distal inflation tube to place said balloon in fluid communication with said distal inflation tube.

11. A catheter as recited in claim 10 wherein said polyetherimide is stronger in compression than in flexure.

12. A catheter as recited in claim 11 wherein said polyetherimide material has a compressive yield strength substantially in the range between 20,000 and 24,000 pounds per square inch (20,000 psi < σ_{c} < 24,000 psi).

13. A catheter as recited in claim 12 wherein said polyetherimide material has a flexural strength substantially in the range between 18,000 and 22,000 pounds per square inch (18,000 psi < σ_{f} < 22,000 psi).

14. A catheter as recited in claim 13 wherein said polyetherimide material has a flexural modulus substantially in the range between 450,000 and 550,000 pounds per square inch (450 ksi < FM < 550 ksi).

15. A catheter as recited in claim 14 where said polyetherimide material is Ultem1000®.

16. A catheter as recited in claim 10 wherein said proximal inflation tube (20) has a proximal region (53), a distal region (48) and an intermediate region (52) therebetween and said proximal end (50) of said distal inflation tube (22) is thermally bonded to said intermediate region (52), said proximal region (53) having a first diameter and said intermediate region (52) having a second diameter, said second diameter being less than said first diameter for maintaining a substantially uniform profile between said proximal inflation tube and said distal inflation tube.

17. A catheter as recited in claim 16 wherein said distal region of said proximal inflation tube has a third diameter, said third diameter being less than said second diameter.

18. A catheter as recited in claim 10 wherein said guidewire tube and said inflatable balloon are made of a respective material, said respective materials for said guidewire tube and for said balloon being thermally compatible with said polyetherimide material and said material for said distal inflation tube.

19. A catheter as recited in claim 18 wherein said materials for said guidewire tube and for said balloon are a nylon polymer and a Polyether Block Amide co-polymer, respectively.

20. A method for integrating a first material with a second material to manufacture an inflation tube for providing both stiffness and flexibility in a balloon catheter, the method which comprises the steps of:
providing a proximal inflation tube having a proximal end and a distal end and being made of a polyetherimide material for providing stiffness for said catheter;
supplying a distal inflation tube having a proximal end and a distal end and being made of said second material for providing flexibility for said catheter, said second material being thermally compatible with said first material; and
thermally bonding said proximal end of said distal inflation tube to said distal end of said proximal inflation tube.

## Patentansprüche

1. Eine Aufblasröhre zum Liefern von Steifigkeit und Flexibilität für einen medizinischen Ballonkatheter, wobei die Aufblasröhre folgende Merkmale aufweist:
ein proximales Segment (20), das ein proximales Ende (36) und ein distales Ende (48) aufweist und mit einem Lumen gebildet ist, wobei das proximale Segment aus einem Polyetherimidmaterial zum Liefern von Steifigkeit für den Katheter hergestellt ist; und
ein distales Segment (22), das ein proximales Ende (50) und ein distales Ende (60) aufweist und das aus einem Material zum Liefern von Flexibilität für den Katheter hergestellt ist, wobei das Material des distalen Segments thermisch kompatibel mit dem Polyetherimidmaterial ist, wobei das proximale Ende des distalen Segments thermisch in Fluidkommunikation mit dem distalen Ende des proximalen Segments verbunden ist.

2. Eine Aufblasröhre gemäß Anspruch 1, bei der das proximale Segment und das distale Segment in Kombination ein Aufblaslumen (90) für die Aufblasröhre bilden, wobei dieselbe ferner folgende Merkmale aufweist:
eine Führungsdrahtröhre (26), die ein proximales Ende und ein distales Ende aufweist, wobei die Führungsdrahtröhre in dem Aufblaslumen positioniert ist, um sich distal von dem distalen Segment zu erstrecken; und
einen aufblasbaren röhrenförmigen Ballon (24), der ein proximales Ende und ein distales Ende aufweist, wobei das distale Ende des Ballons an dem distalen Ende der Führungsdrahtröhre angebracht ist, wobei das proximale Ende des Ballons an dem distalen Ende des distalen Segments angebracht ist, um den Ballon in Fluidkommunikation mit der Aufblasröhre zu plazieren.

3. Eine Aufblasröhre gemäß Anspruch 1, bei der das Polyetherimidmaterial in Kompression fester ist als in Biegung.

4. Eine Aufblasröhre gemäß Anspruch 1, bei der das Polyetherimidmaterial eine Druckstreckgrenze im wesentlichen im Bereich zwischen 20.000 und 24.000 Pfund pro Quadratzoll (20.000 psi < σ_{c} < 24.000 psi) aufweist.

5. Eine Aufblasröhre gemäß Anspruch 4, bei der das Polyetherimidmaterial eine Biegefestigkeit im wesentlichen im Bereich zwischen 18.000 und 22.000 Pfund pro Quadratzoll (18.000 psi < σ_{f} < 22.000 psi) aufweist.

6. Eine Aufblasröhre gemäß Anspruch 5, bei der das Polyetherimidmaterial ein Biegemodul im wesentlichen im Bereich zwischen 450.000 und 550.000 Pfund pro Quadratzoll (400 ksi < FM < 550 ksi) aufweist.

7. Eine Aufblasröhre gemäß Anspruch 6, bei der das PEI-Material Ultem1000® ist.

8. Eine Aufblasröhre gemäß Anspruch 2, bei der die jeweilige Führungsdrahtröhre und der Ballon aus jeweiligen Materialien hergestellt sind, wobei die jeweiligen Materialien für die Führungsdrahtröhre und für den Ballon thermisch kompatibel mit dem Polyetherimidmaterial und mit dem Material für das distale Segment sind.

9. Eine Aufblasröhre gemäß Anspruch 8, bei der der Ballon thermisch mit der Führungsdrahtröhre und mit dem distalen Ende des distalen Segments verbunden ist.

10. Ein medizinischer Ballonkatheter, der folgende Merkmale aufweist:
eine proximale Aufblasröhre (20), die ein proximales Ende (36) und ein distales Ende (48) aufweist und die aus einem Polyetherimidmaterial zum Liefern von Steifigkeit für den Katheter hergestellt ist;
eine distale Aufblasröhre (22), die aus einem Material zum Liefern von Flexibilität in dem Katheter hergestellt ist und die ein proximales Ende (50) und ein distales Ende (60) aufweist, wobei das Material für die distale Aufblasröhre thermisch kompatibel mit dem Polyetherimidmaterial ist, wobei das proximale Ende der distalen Aufblasröhre thermisch in Fluidkommunikation mit dem distalen Ende der proximalen Aufblasröhre verbunden ist, um ein Aufblaslumen zu bilden;
eine Führungsdrahtröhre (26), die ein proximales Ende und ein distales Ende aufweist und mit einem Führungsdrahtlumen (68) gebildet ist, wobei die Führungsdrahtröhre in dem Aufblaslumen positioniert ist, um sich distal von der distalen Aufblasröhre zu erstrecken; und
einen aufblasbaren röhrenförmigen Ballon (24), der ein proximales Ende und ein distales Ende aufweist, wobei das distale Ende des Ballons an dem distalen Ende der Führungsdrahtröhre angebracht ist und das proximale Ende des Ballons an dem distalen Ende der distalen Aufblasröhre angebracht ist, um den Ballon in Fluidkommunikation mit der distalen Aufblasröhre zu plazieren.

11. Ein Katheter gemäß Anspruch 10, bei dem das Polyetherimid in Kompression fester ist als in Biegung.

12. Ein Katheter gemäß Anspruch 11, bei dem das Polyetherimidmaterial eine Druckstreckgrenze im wesentlichen im Bereich zwischen 20.000 und 24.000 Pfund pro Quadratzoll (20.000 psi < σ_{c} < 24.000 psi) aufweist.

13. Ein Katheter gemäß Anspruch 12, bei dem das Polyetherimidmaterial eine Biegefestigkeit im wesentlichen im Bereich zwischen 18.000 und 22.000 Pfund pro Quadratzoll (18.000 psi < σ_{f} < 22.000 psi) aufweist.

14. Ein Katheter gemäß Anspruch 13, bei dem das Polyetherimidmaterial einen Biegemodus im wesentlichen im Bereich zwischen 450.000 und 550.000 Pfund pro Quadratzoll (450 ksi < FM < 550 ksi) aufweist.

15. Ein Katheter gemäß Anspruch 14, bei dem das Polyetherimidmaterial Ultem1000® ist.

16. Ein Katheter gemäß Anspruch 10, bei dem die proximale Aufblasröhre (20) eine proximale Region (53), eine distale Region (48) und eine Zwischenregion (52) zwischen denselben aufweist und das proximale Ende (50) der distalen Aufblasröhre (22) thermisch mit der Zwischenregion (52) verbunden ist, wobei die proximale Region (53) einen ersten Durchmesser aufweist und die Zwischenregion (52) einen zweiten Durchmesser aufweist, wobei der zweite Durchmesser geringer ist als der erste Durchmessern, zum Beibehalten eines im wesentlichen einheitlichen Profils zwischen der proximalen Aufblasröhre und der distalen Aufblasröhre.

17. Ein Katheter gemäß Anspruch 16, bei dem die distale Region der proximalen Aufblasröhre einen dritten Durchmesser aufweist, wobei der dritte Durchmesser geringer ist als der zweite Durchmesser.

18. Ein Katheter gemäß Anspruch 10, bei dem die Führungsdrahtröhre und der aufblasbare Ballon aus einem jeweiligen Material hergestellt sind, wobei die jeweiligen Materialien für die Führungsdrahtröhre und für den Ballon thermisch kompatibel mit dem Polyetherimidmaterial und dem Material für die distale Aufblasröhre sind.

19. Ein Katheter gemäß Anspruch 18, bei dem die Materialien für die Führungsdrahtröhre und für den Ballon ein Nylonpolymer bzw. ein Polyether-Block-Amid-Copolymer sind.

20. Ein Verfahren zum Integrieren eines ersten Materials mit einem zweiten Material, um eine Aufblasröhre zum Liefern von sowohl Steifigkeit als auch Flexibilität in einem Ballonkatheter herzustellen, wobei das Verfahren folgende Schritte aufweist:
Liefern einer proximalen Aufblasröhre, die ein proximales Ende und ein distales Ende aufweist und die aus einem Polyetherimidmaterial zum Liefern von Steifigkeit für den Katheter hergestellt ist;
Liefern einer distalen Aufblasröhre, die ein proximales Ende und ein distales Ende aufweist und aus dem zweiten Material hergestellt ist, zum Bereitstellen von Flexibilität für den Katheter, wobei das zweite Material thermisch mit dem ersten Material kompatibel ist; und
thermisches Verbinden des proximalen Endes der distalen Aufblasröhre mit dem distalen Ende der proximalen Aufblasröhre.

## Revendications

1. Tube de gonflage destiné à fournir une rigidité et une flexibilité à un cathéter à ballonnet médical, ledit tube de gonflage comprenant :
un segment proximal (20) comportant une extrémité proximale (36) et une extrémité distale (48) et étant muni d'une lumière, ledit segment proximal étant constitué d'un matériau de polyétherimide destiné à fournir une rigidité audit cathéter, et
un segment distal (22) comportant une extrémité proximale (50) et une extrémité distale (60) et étant constitué d'un matériau destiné à fournir une flexibilité audit cathéter, ledit matériau dudit segment distal étant thermiquement compatible avec ledit matériau de polyétherimide, ladite extrémité proximale dudit segment distal étant liée thermiquement en communication de fluide à ladite extrémité distale dudit segment proximal.

2. Tube de gonflage selon la revendication 1, dans lequel ledit segment proximal et ledit segment distal, en combinaison, établissent une lumière de gonflage (90) pour ledit tube de gonflage et comprenant en outre :
un tube de guide souple (26) comportant une extrémité proximale et une extrémité distale, ledit tube de guidage de fil étant positionné dans ladite lumière de gonflage pour s'étendre de façon distale par rapport audit segment distal, et
un ballonnet tubulaire gonflable (24) comportant une extrémité proximale et une extrémité distale, ladite extrémité distale dudit ballonnet étant fixée à ladite extrémité distale dudit tube de guide souple, ladite extrémité proximale dudit ballonnet étant fixée à ladite extrémité distale dudit segment distal pour placer ledit ballonnet en communication de fluide avec ledit tube de gonflage.

3. Tube de gonflage selon la revendication 1, dans lequel ledit matériau de polyétherimide est plus résistant en compression qu'en flexion.

4. Tube de gonflage selon la revendication 1, dans lequel ledit matériau de polyétherimide présente une limite d'élasticité à la compression pratiquement dans la plage entre 20 000 et 24 000 livres par pouce carré (20 000 psi < σ_{c} < 24 000 psi).

5. Tube de gonflage selon la revendication 4, dans lequel ledit matériau de polyétherimide présente une résistance à la flexion pratiquement dans la plage entre 18 000 et 22 000 livres par pouce carré (18 000 psi < σ_{f} < 22 000 psi).

6. Tube de gonflage selon la revendication 5, dans lequel ledit matériau de polyétherimide présente un module de flexion pratiquement dans la plage entre 450 000 et 550 000 livres par pouce carré (400 ksi < FM < 550 ksi).

7. Tube de gonflage selon la revendication 6, dans lequel ledit matériau de polyétherimide est du Ultem1000®.

8. Tube de gonflage selon la revendication 2, dans lequel le tube de guide souple respectif et ledit ballonnet sont constitués de matériaux respectifs, lesdits matériaux respectifs pour ledit tube de guide souple et pour ledit ballonnet étant thermiquement compatibles avec ledit matériau de polyétherimide et avec ledit matériau destiné audit segment distal.

9. Tube de gonflage selon la revendication 8, dans lequel ledit ballonnet est thermiquement lié audit tube de guide souple et à ladite extrémité distale dudit segment distal.

10. Cathéter à ballonnet médical comprenant :
un tube de gonflage proximal (20) comportant une extrémité proximale (36) et une extrémité distale (48) et étant réalisé à partir d'un matériau de polyétherimide en vue de fournir une rigidité audit cathéter,
un tube de gonflage distal (22) constitué d'un matériau destiné à fournir une flexibilité audit cathéter et comportant une extrémité proximale (50) et une extrémité distale (60), ledit matériau destiné audit tube de gonflage distal étant thermiquement compatible avec ledit matériau de polyétherimide, ladite extrémité proximale dudit tube de gonflage distal étant thermiquement liée en communication de fluide à ladite extrémité distale dudit tube de gonflage proximal pour établir une lumière de gonflage,
un tube de guide souple (26) comportant une extrémité proximale et une extrémité distale et étant muni d'une lumière de guide souple (68), ledit tube de guide souple étant positionné dans ladite lumière de gonflage pour s'étendre de façon distale par rapport audit tube de gonflage distal, et
un ballonnet tubulaire gonflable (24) comportant une extrémité proximale et une extrémité distale, ladite extrémité distale dudit ballonnet étant fixée à ladite extrémité distale dudit tube de guide souple, ladite extrémité proximale dudit ballonnet étant fixée à ladite extrémité distale dudit tube de gonflage distal pour placer ledit ballonnet en communication de fluide avec ledit tube de gonflage distal.

11. Cathéter selon la revendication 10, dans lequel ledit polyétherimide est plus résistant en compression qu'en flexion.

12. Cathéter selon la revendication 11, dans lequel ledit matériau de polyétherimide présente une limite d'élasticité à la compression pratiquement dans la plage entre 20 000 et 24 000 livres par pouce carré (20 000 psi < σ_{c} < 24 000 psi).

13. Cathéter selon la revendication 12, dans lequel ledit matériau de polyétherimide présente une résistance à la flexion pratiquement dans la plage entre 18 000 et 22 000 livres par pouce carré (18 000 psi < σ_{f} < 22 000 psi).

14. Cathéter selon la revendication 13, dans lequel ledit matériau de polyétherimide présente un module de flexion pratiquement dans la plage 450 000 et 550 000 livres par pouce carré (450 ksi < FM < 550 ksi).

15. Cathéter selon la revendication 14, où ledit matériau de polyétherimide est du Ultem 1000®.

16. Cathéter selon la revendication 10, dans lequel ledit tube de gonflage proximal (20) comporte une région proximale (53), une région distale (48) et une région intermédiaire (52) entre celles-ci et ladite extrémité proximale (50) dudit tube de gonflage distal (22) est thermiquement liée à ladite région intermédiaire (52), ladite région proximale (53) présentant un premier diamètre et ladite région intermédiaire (52) présentant un second diamètre, ledit second diamètre étant inférieur audit premier diamètre en vue de maintenir un profil pratiquement uniforme entre ledit tube de gonflage proximal et ledit tube de gonflage distal.

17. Cathéter selon la revendication 16, dans lequel ladite région distale dudit tube de gonflage proximal présente un troisième diamètre, ledit troisième diamètre étant inférieur audit second diamètre.

18. Cathéter selon la revendication 10, dans lequel ledit tube de guide souple et ledit ballonnet gonflable sont constitués d'un matériau respectif, ledit matériau respectif avec ledit tube de guide souple et pour ledit ballonnet étant thermiquement compatible avec ledit matériau de polyétherimide et avec ledit matériau pour ledit tube de gonflage distal.

19. Cathéter selon la revendication 18, dans lequel ledit matériau pour ledit tube de guide souple et pour ledit ballonnet sont un polymère de nylon et un copolymère polyéther séquencé-amide, respectivement.

20. Procédé d'intégration d'un premier matériau à un second matériau pour fabriquer un tube de gonflage destiné à fournir à la fois une rigidité et une flexibilité dans un cathéter à ballonnet, le procédé comprenant les étapes consistant à :
réaliser un tube de gonflage proximal comportant une extrémité proximale et une extrémité distale et étant constitué d'un matériau de polyétherimide en vue de fournir une rigidité audit cathéter,
fournir un tube de gonflage distal comportant une extrémité proximale et une extrémité distale et étant constitué dudit second matériau destiné à fournir une flexibilité audit cathéter, ledit second matériau étant thermiquement compatible avec ledit premier matériau, et
lier thermiquement ladite extrémité proximale dudit tube de gonflage distal à ladite extrémité distale dudit tube de gonflage proximal.
